# EUROPEAN PATENT APPLICATION

(11) **EP 2 826 768 A1**
(43) Date of publication of application: **21.01.2015**
(21) Application number: 13176542.2
(22) Date of filing: 15.07.2013
(51) Int. Cl.: C07C 253/30, C07C 255/51

(54) **Process for manufacturing 1,2,3-triylidenetris(cyanomethanylylidene)tris(2,3,5,6-tetrafluorobenzonitrile)-cyclopropane**

(71) Applicant: Novaled GmbH, 01307 Dresden (DE)
(72) Inventor: Dorok, Sascha, 01099 Dresden (DE); Wutke, Jens, 01159 Dresden (DE); Klose, Manuela, 01465 Dresden (DE); Brothagen, Julia, 01067 Dresden (DE)
(74) Representative: Bittner, Thomas L.

(57) **Abstract**

The present invention relates to industrial process for manufacturing 1,2,3-triylidenetris(cyanomethanylylidene))tris(2,3,5,5-tetrafluorobenzonitrile)-cyclopropane by oxidizing the precursor methyl idenecyclopropene compound (c) with concentrated nitric acid.

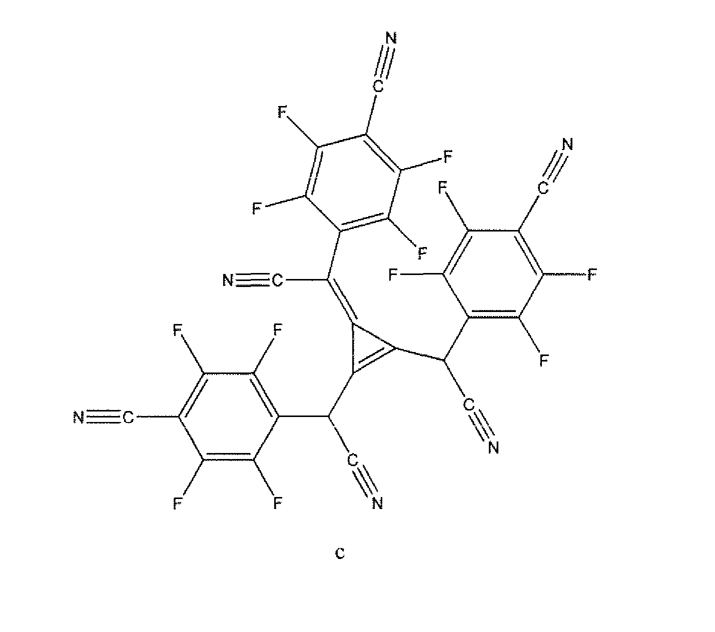

## Description

The present invention relates to manufacturing process for 1,2,3-triylidenetris(cyanomethanylylidene))tris(2,3,5,6-tetrafluorobenzonitrile)-cyclopropane.

The title [3]-radialene compound has been found to be specifically useful as p-dopant for common OLED hole transport materials. Its preparation from tetrachlorocyclopropene and 4-(cyanomethyl)-2,3,5,6-tetrafluorabenzonitrile was first described in US 8 057 712 B2, herein incorporated as a reference. For an industrial production, this state-of-the-art process has some drawbacks. First of all, the known oxidation procedure using the highly corrosive mixture composed of concentrated nitric and hydrobromic acid is suboptimal from the industrial hygiene viewpoint and difficult to be scaled up reproducibly. Moreover, perchlorocyclopropene is an expensive starting material.

It is therefore an object of the present invention to provide a robust process applicable in the industrial scale.

### Summary of the invention

The object is achieved by a process comprising the step of oxidizing 1,2-bis[cyano-(4-cyano-2,3,5,6-tetrafluorphenyl)methyl]-3-[cyano-(4-cyano-2,3,5,6-tetrafluorphenyl)methylidene]-cyclopropene (c) 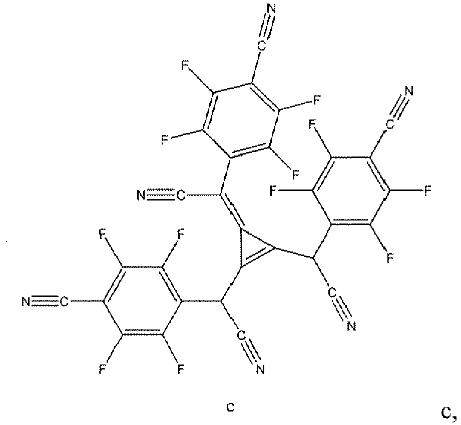 wherein the oxidizing is carried out with nitric acid.

It is preferred that in this oxidizing step, compound (c) is added to nitric acid. Preferably, the oxidation is carried out in nitric acid as a solvent or in a mixed solvent consisting of nitric acid and organic acid. Preferred organic acid is acetic acid. Preferably, a solution of compound (c) in organic acid is added to nitric acid. Preferably, starting concentration of nitric acid is above 30 % by weight (wt.%), more preferably above 60 wt.%, most preferably above 90 wt. %. Preferred reaction temperature is in the range 10-70 °C, more preferably in the range 25-55 °C, most preferably in the range 35-45 °C.

Preferably, compound (c) is prepared in one step from pentachlorocyclopropane and (4-cyano-2,3,5,6-tetrafluorphenyl)acetonitrile, preferably in presence of a strong inorganic base. Preferred solvent for this reaction is tetrahydrofuran. Preferred base is lithium hydride. Preferred reaction temperature is in the range -30-+60 °C, more preferably in the range -15-+45 °C, most preferably in the range 0-30 °C.

It is further preferred that (4-cyano-2,3,5,6-tetrafluorphenyl)acetonitrile is prepared in one step from potassium salt of 2-[4-cyano-(2,3,5,6-tetrafluorophenyl)]-cyanoacetic acid ethyl ester (a)

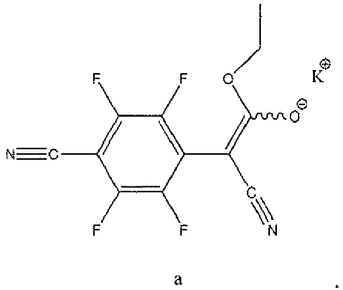

Preferably, this reaction is carried out in presence of at least partially soluble calcium salt. Preferred solvent for the (4-cyano-2,3,5,6-tetrafluorphenyl)acetonitrile preparation is acetic acid that may contain up to 60 wt. % water. Preferred calcium salt is calcium acetate. Preferred temperature range is 60-140 °C, more preferably 80-120 °C, most preferred embodiment is carrying out this reaction at reflux temperature of aqueous acetic acid at ordinary pressure.

It is further preferred that potassium salt (a) is prepared in one step from cyanoacetic acid ethyl ester and pentafluorobenzonitrile. Preferably, this reaction is carried out in presence of anhydrous potassium carbonate. Preferred solvent for this reaction is acetonitrile. Preferred temperature range is 40-120 °C, more preferably 60-100 °C, most preferred embodiment is carrying out this reaction at reflux temperature of acetonitrile at ordinary pressure.

### Detailed description of the invention

During the development of the industrial scale process, it was surprisingly found that most reproducible results in the last oxidation step can be obtained when using nitric acid as both the solvent and oxidizing agent, many other combinations of common industrial oxidants and solvents failed in attempts to obtain stable yields and constant quality of the product.

In the preferred embodiment, pentachlorocyclopropane has successfully replaced tetrachlorocyclopropene. It is supposed that tetrachlorocyclopropene is still the intermediate in this variant of the present process, however, if it is formed in situ during the reaction, it is supposed that it is practically immediately consumed in subsequent reactions leading finally to the dilithium salt of the compound (c).

Compound (c) in its neutral form may be obtained from the dilithium salt by acidification with an organic acid like acetic acid. It was found that it is not necessary to isolate the salt or neutral form of the compound (c). In the preferred embodiment, a solution of compound (c) in acetic acid is used directly in the last step.

Claisen alkylation of cyanoacetic acid ethyl ester may be carried out in various polar aprotic solvents, acetonitrile was found superior. Potassium carbonate is an advantageous base in this step, allowing isolation of the product as a crystalline potassium salt. It was found that the salt comprises traces of fluoride which can evolve undesired toxic and corrosive hydrogen fluoride in the next acidic hydrolysis and decarboxylation advantageously carried out in one pot reaction. It was proven that addition of at least partially soluble calcium salt binds fluoride as insoluble calcium salt despite the acidic pH and allows quick and efficient conversion in the desired intermediate 4-(cyanomethyl)-2,3,5,6-tetrafluorobenzonitrile (b) without necessity for a laborious removing of the fluoride traces from the starting potassium salt.

### Examples

### Step 1: potassium 2-cyano-2-(4-cyano-2,3,5,6-tetrafluorophenyl)-1-ethoxyethen-1-olate (a)

Potassium carbonate (20.0 kg, 145 mol, 1.2 eq) was placed in the reactor, followed by inerting with nitrogen. 2,3,4,5,6-pentafluorobenzonitrile (23.5 kg, 122 mol, 1.0 eq) and acetonitrile (220 L) were added and the mixture was stirred at 50 rounds per minute (RPM). Ethyl 2-cyanoacetate (14.0 kg, 124 mol, 1.0 eq) was added followed by another 35 L acetonitrile. The mixture was heated to reflux (82 °C) and allowed to react over 17 hours until HPLC indicated complete conversion of starting material (< 1 % of the starting concentration). The mixture was cooled down to +55 °C and vacuum-filtered using a heated Halarfilter (stringed with BECO steril). The reactor and the filter residue were flushed with 30 L acetonitrile and the filter was subsequently vigorously flushed with nitrogen. The filtrate was led back to the purified (with boiling acetone), dried and inertized reactor and concentrated *in vacuo* at 60-80 °C to a residue amount of acetonitrile of about 22 kg. For each kilogram of acetonitrile, 0.5 kg n-heptane (11 kg) and 1.2 kg toluene (26.4 kg) were added, subsequently. The suspension was cooled to 0-5 °C and stirred over 60 minutes. The precipitate was isolated using a BECO steril-stringed Halarfilter (F906) and the reactor and the filter residue were flushed with a mixture toluene (16.4 kg) / heptane (11 kg). The filter residue was dried in vacuum at 40 °C until dry content was > 99.8 wt.%. The desired compound (a) was obtained in 99 % (38.9 kg) yield.

### Step 2: 4-(cyanomethyl)-2,3,5,6-tetrafluorobenzonitrile (b)

Calcium acetate monohydrate (ca. 0.12 kg/kg (a); 5.0 kg, 28 mol, 0.2 eq) was diluted in water (ca. 1.85kg/kg (a); 60 kg) within 10 minutes. Potassium 2-cyano-2-(4-cyano-2,3,5,6-tetrafluorophenyl)-1-ethoxyethen-1-olate (a) (38.9 kg, 120 mol, 1.0 eq) was added to the stirred solution (80 RPM), followed by glacial acetic acid (ca. 1.85kg/kg (a); 72 kg). After complete dissolving of the potassium salt, sulfuric acid (ca. 60 g/kg (a), 2.4 kg, 24 mol, 0.2 eq) was added and the mixture was heated to reflux over 30 minutes and kept refluxing over 5.5 hours until HPLC indicated complete conversion of starting material (< 1 % of the starting concentration). The mixture was cooled down to room temperature and ice water (80 kg) was added. The organic layer was separated and the aqueous layer was extracted twice with toluene (2x40 kg). The combined organic layers were filtered using a Halarfilter (stringed with BECO steril) to remove calcium fluoride co-precipitated with the formed calcium sulfate and the filtrate was led back to the water-flushed reactor and washed with water (60 kg) and 10% aqueous sodium bicarbonate solution (60 kg), successively. Afterwards, the organic layer was concentrated *in vacuo* at 60-80 °C over 13 hours to dryness and 2-propanol (16 kg) was added to the oily/partly crystallized residue; the solid dissolved again. 20 kg water were added and the product was allowed to precipitate over at least 1 hour at 0-5 °C. The suspension was filtered through a BECO steril-stringed pre-cooled Halarfilter and the reactor as well as the filter residue were flushed with 10 kg of 2-propanol/water mixture (volume ratio 1:1). The product was dried under slightly lowered pressure (it melts at 40-50°C and has a tendency to sublime) until dry content was > 99.9 wt.%. The desired compound (b) was obtained in 64 % (16.4 kg) yield.

### Step 3: 4,4'-((3-(cyano(4-cyano-2,3,5,6-tetrafluorophenyl)methylene)cycloprop-1-ene-1,2-diyl)bis(cyanomethylene))bis(2,3,5,6-tetrafluorobenzonitrile) (c)

After the reactor was inertized with nitrogen, lithium hydride (0.80 kg, 101 mol, 9.8 eq) was suspended in 8 kg dry THF without stabilizers. The reactor was flushed with nitrogen (200 L/h) and further 88 kg THF were added to the suspension stirred at 100 RPM. After cooling down to 10-15 °C, a solution of 4-(cyanomethyl)-2,3,5,6-tetrafluorobenzonitrile (b) (8.4 kg, 39 mol, 3.8 eq) in 25 kg THF was added over 45 minutes, maintaining the temperature below 15 °C. After complete addition and stirring over further 45 minute period, a solution of pentachlorocyclopropane (2.2 kg, 10 mol, 1.0 eq) in 6 kg THF was added at 0-5 °C over 30 minutes. The addition vessel was subsequently rinsed with 3 kg THF. The reaction mixture was allowed to warm to room temperature over a 42 hour period. 50 kg water were added carefully (initially, 2 kg to quench the unreacted LiH) and the mixture was acidified with 5 kg aqueous hydrochloric acid (concentration 36-38 wt.%). 40 kg ethyl acetate were added and, after stirring over 30 minutes and allowing the layers to separate over another 30 minute period, the layers were separated. The aqueous layer was extracted in similar manner a second time (with 20 kg ethyl acetate, 15 minutes stirring). The combined organic layers were led back to the water-damp reactor and washed with brine (2x62 kg) and with 10 wt.% aqueous sodium bicarbonate solution (2x65 kg), successively. Afterwards, the organic layer was evaporated to dryness *in vacuo* at 40-60 °C over 13 hours. Glacial acetic acid (150 kg) was added to the remaining residue and nearly 2/3 were removed again at 40°C by means of vacuum distillation. The glacial acetic acid solution of the product (overall weight 59.7 kg) was cooled down to room temperature and directly used in the next step.

### Step 4: 4,4',4"-((1E,1'E,1"E)-cylopropane-1,2,3-triylidenetris(cyanomethanylylidene))tris (2,3,5,6-tetrafluorobenzonitrile) (d)

Nitric acid (99 wt.%, 94 kg) was placed in the reactor and the glacial acetic acid solution of 4,4'-((3-(cyano(4-cyano-2,3,5,6-tetrafluorophenyl)methylene)cycloprop-1-ene-1,2-diyl)bis-(cyanomethylene))bis(2,3,5,6-tetrafluorobenzonitrile) (c) from the previous step (total weight 56.2 kg) was added over 30 minutes to the stirred acid, maintaining the temperature < 40 °C. The addition vessel was subsequently rinsed with 3 kg glacial acetic acid. The mixture was cooled down to +20 °C with a cold water jacket and 115 kg water were added in portions, maintaining the temperature between 20-30 °C. The product was allowed to precipitate over further 30 minutes under stirring. The suspension was filtered using a Halarfilter and the filter residue was dried in a nitrogen stream over 2 hours. The crude product was washed by suspending in water (2x40 kg, stirring over 10 min, isolation by filtration on BECO Endura-stringed Halarfilter) and dried in a nitrogen stream (3 hours), followed by drying in vacuum at 40 °C (48 hours). The crude radialene compound (6.4 kg) was washed by suspending with 10 kg acetonitril, filtered again and dried *in vacuo* at 40 °C overnight. The remaining 3.189 kg were re-crystallized from 35 kg acetonitrile to give finally 1.845 kg of the desired pure compound (d) (28.4 % over 2 steps). If the described reaction started with 65 wt.% aqueous nitric acid instead 99 wt. %, the reaction took 3 hours and the final yield of the purified product over two steps was 25 %.

The features of the invention disclosed in the previous description and in the claims can be essential individually as well as in any combination for the realization of the invention in its various embodiments.

## Claims

1. Process for manufacturing 1,2,3-triylidenetris(cyanomethanylylidene))tris(2,3,5,6-tetrafluorobenzonitrile)-cyclopropane comprising the step of oxidizing 1,2-bis[cyano-(4-cyano-2,3,5,6-tetrafluorphenyl)methyl]-3-[cyano-(4-cyano-2,3,5,6-tetrafluorphenyl)methylidene]-cyclopropene (c) 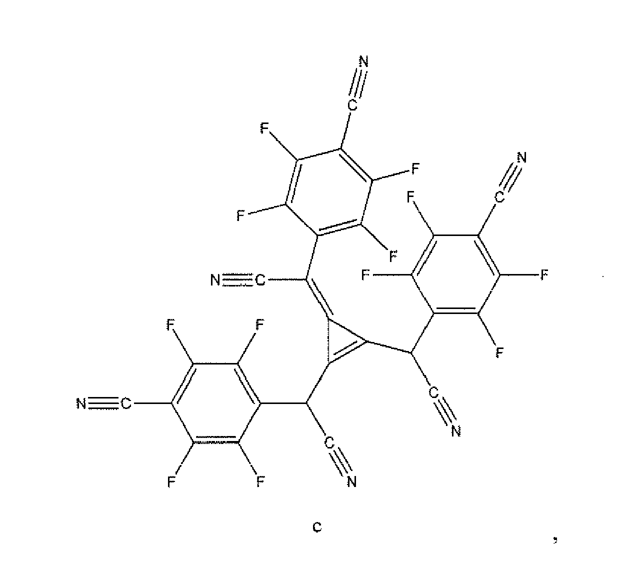 wherein the oxidizing is carried out with nitric acid.

2. Process according to claim 1, wherein the oxidation is carried out in nitric acid or in a solvent consisting of nitric and organic acid.

3. Process according to claim 1 or 2, wherein compound (c) is added to nitric acid.

4. Process according to any of claims 1-3, wherein compound (c) is prepared in one step from pentachlorocyclopropane and (4-cyano-2,3,5,6-tetrafluorphenyl)acetonitrile.

5. Process according to claim 4, wherein the reaction is carried out in tetrahydrofuran in presence of a base, preferably a strong base.

6. Process according to claim 5, wherein the base is lithium hydride.

7. Process according to any of claims 4-6, wherein (4-cyano-2,3,5,6-tetrafluorphenyl)acetonitrile is prepared in one step from potassium salt of 2-[4-cyano-(2,3,5,6-tetrafluorophenyl)]-cyanoacetic acid ethyl ester (a)

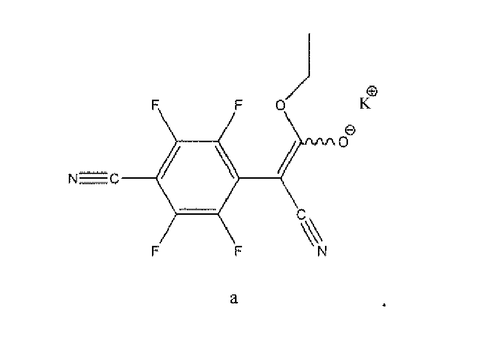

8. Process according to claim 7, wherein the reaction is carried out in presence of at least partially soluble calcium salt.

9. Process according to claim 7 or 8, wherein the reaction is carried out in acetic acid.

10. Process according to any of claims 7-9, wherein potassium salt (a) is prepared in one step from cyanoacetic acid ethyl ester and pentafluorobenzonitrile.
